# EUROPEAN PATENT SPECIFICATION

(11) **EP 2 026 664 B1**
(45) Date of publication and mention of the grant of the patent: **10.09.2014**
(21) Application number: 07735750.7
(22) Date of filing: 03.05.2007
(51) Int. Cl.: A23L 1/00, A23L 1/22, A23P 1/04

(54) **ONE STEP SPRAY-DRYING PROCESS**
EINSTUFIGES SPRÜHTROCKNUNGSVERFAHREN
PROCÉDÉ DE SÉCHAGE PAR ATOMISATION EN UNE SEULE ÉTAPE

(30) Priority: 19.05.2006 WO PCT/IB2006/051606
(43) Date of publication of application: 25.02.2009
(73) Proprietor: Firmenich S.A., 1211 Geneva 8 (CH)
(72) Inventor: TROPHARDY, Gil, F-01170 Gex (FR)
(74) Representative: Dale, Gavin Christopher
(86) International application number: PCT/IB2007/051655
(87) International publication number: WO 2007/135583

(56) References cited:
- WO-A-2004/062382
- FR-A- 2 758 055
- US-A- 4 634 598
- US-A- 5 607 708
- US-B1- 6 482 433
- JACKSON L S ET AL: "MICROENCAPSULATION AND THE FOOD INDUSTRY" FOOD SCIENCE AND TECHNOLOGY, ACADEMIC PRESS, US, vol. 24, 1991, pages 289-297, XP001009807 ISSN: 0023-6438
- SOOTTITANTAWAT A ET AL: "Influence of emulsion and powder size on the stability of encapsulated d-limonene by spray drying" INNOVATIVE FOOD SCIENCE AND EMERGING TECHNOLOGIES, ELSEVIER, AMSTERDAM, NL, vol. 6, no. 1, March 2005 (2005-03), pages 107-114, XP004914127 ISSN: 1466-8564

## Description

### Technical Field

The present invention relates to a method for preparing capsules having an average diameter in the range of 100 to 2000 µm encapsulating an active ingredient, namely a flavour, fragrance, a functional additive such as a food or nutrition ingredient, an insect repellent or attractant, a pesticide, an antimicrobial or antibacterial agent, dye or antioxidant ingredients, or yet appropriate mixtures of one or more of such ingredients.

The invention further relates to capsules as such, a method for controlling the water dissolution properties of sprayed particles containing an active ingredient and having an average diameter in the range of 100 to 2000 µm, and the use of a multi stage drying apparatus for preparing capsules of the invention.

### Background of the Invention and Problem to be Solved

The objective of the present invention is the encapsulation of active compounds and/or compositions in particles having an average diameter of 100 to 2000 µm. Encapsulation, in general, has the purpose of providing a stable, easy to use and process, and transportable, form of active ingredients. If the active ingredient is liquid at ambient temperature, encapsulation within particles has the further advantage of easier handling and mixing with dry products and providing a less concentrated form of the active ingredient, the latter being an advantage in some instances.

Encapsulation in free flowing powders generally has the purpose of creating a form of active ingredients that can be conveniently dosed and mixed with other ingredients or be directly added to consumer end products. In this regard, particle size and load of active ingredient become relevant. Relatively large particles become useless if small quantities of active ingredient are to be added to a consumer end product, because the particle distribution in the product may bring about a significant fluctuation of final concentration in different regions thereof. Relatively small particles, on the other hand, suffer from a less beneficial surface to volume ratio. This ratio is particularly relevant with volatile active ingredients, such as many fragrances and flavours, where loss through evaporation needs to be prevented. Furthermore, the smaller the particles, the higher the risk of explosion of the powder and handling has accordingly to be done more carefully and at a higher cost.

Spray drying has been widely used to encapsulate active ingredients, in particular flavours and/or fragrances, in a glassy matrix and is substantially disclosed in the prior art. In such a process the active ingredient is dissolved or finely dispersed in a water medium comprising a carrier substance susceptible of forming a matrix capable of encapsulating the active ingredient and optionally other ingredients such as emulsifiers, dyes, etc, and this mixture, commonly known as the "feed", is finely sprayed into a chamber where the formed droplets are put in contact with a hot gas, air or nitrogen for example. Water is then evaporated very quickly from the droplets and dry particles ranging from 5 to a couple hundred microns are recovered. These powders contain the active substance finely dispersed in the dry film forming or polymer matrix.

Typically, the drops of the suspension are sprayed through a nozzle in a spraying tower where they are exposed to the hot air, causing the evaporation of water and thus formation of dried particles that are generally collected at the bottom of the drying tower. However, typical spray-drying is not suitable to produce capsules of much higher than about 100 µ diameter, because drying of these large drops would not be sufficiently quick to prevent sticking of the drops to the inner wall of the tower. A further drawback of traditional spray drying methods is the fact that the viscosity of the suspension or emulsion to be spray dried needs to be kept to a few hundred mPas, in order for it to be finely atomized. This restricts the amount of substances such as thickeners that can be added to the suspension or emulsion and/or requires addition of substantial amounts of water. The latter must be removed during the drying step, thus increasing production costs.

Conventional spray drying is therefore not suitable for producing, in one step, particles having a particle diameter in the range of 100 - 2000 µ. If large particles are desired, further steps are needed. In multistage dryers for example, a fluidised bed is integrated at the bottom of the spraying chamber and the fine droplets (less than 150 microns) produced from the nozzle or a rotary atomizer are agglomerated in the chamber to yield agglomerates. These agglomerates are collected in the fluidised bed to finish their drying. The product is then composed of agglomerates of finer primary particles and the maximum size of these agglomerates cannot be larger than 400 microns.

In order to produce big granules of active ingredients or compositions, it is also known to add anti-caking agents into the spray tower during the spraying of the emulsion or suspension. For example, in WO 04/062382 there is disclosed a process for producing beadlet preparations of fat-soluble substances, wherein an aqueous suspension of a fat-soluble substance is fed into a spray tower while simultaneously introducing powder starch and hot air through separate inlets. This process has a temperature of up to 200°C, but preferably 60-120°C in the spray zone, and the fat-soluble substance is a vitamin, such as vitamins A, D, E, K, a carotenoid, a polyunsaturated fatty acid, an oil or a fat.

WO 91/17821 discloses a process for preparing microcapsules containing a flavourant embedded in a matrix material by spraying an aqueous suspension under the supply of air having a temperature of 50 to 120°C, while simultaneously introducing a spraying agent. The spraying agent may comprise starch, modified starch, tri-calcium phosphate and others. Some examples of this document clearly show that there is a substantial loss of up to 50 wt % of the flavourant material present in the initial emulsion (Examples 1, 3 and 4).

US 4,870,196 describes a method of preparing powdered, free-flowing tocopheryl succinate, wherein a powdering agent of said tocopheryl succinate is present in the spraying zone to facilitate the formation of droplets. The powdering agent is said to "settle" on the tocopheryl succinate as a thin layer thus increasing the size of the particles.

US 4,764,317 discloses a process which includes the production of wet beads that fall onto a layer of powder preventing the beads from sticking together or onto the equipment walls. US 6,482,433 B1 discloses a process for preparing capsules using spray-dying.

Finally, the general idea of using anti-caking agents such as starch to produce large granules of vitamins for example is discussed in the International Encyclopaedia of Food Nutrition, Volume 9, Fat-soluble vitamins, 1970, for example.

In all spray drying or multistage drying processes of this type, the anti-caking agent is added to form a non-reactive coating around the droplets of feed. The size of the feed droplets is increased by a layer of anti-caking agent, but the encapsulating matrix of film-forming or polymer carrier is not modified by this coating.

Therefore, if one desires to obtain particles with slower dissolution rate in aqueous media, a further process must be added in a second step to coat the particles for instance with a wax or a fat to delay their solubility, or to provoke reaction of the particles react with chemical ingredients susceptible of modifying the matrix.

The aim of the present invention is to provide a more cost-effective spraying process, in particular a spray-drying process, wherein a single step allows the production of large particles (between 100 and 2000µm size) of active ingredient having properties that, as regards dissolution rate and release of the active ingredient, are adapted to application needs. To this effect, the invention provides a one step effective process wherein the matrix is chemically or physically modified during spray drying or spray cooling of the emulsion or suspension of the active/carrier mixture thanks to the addition of a powdering agent, together with a solid or liquid substance susceptible of inducing modification of the carrier matrix, particularly gellation, crystallization, cross-linking or polymerisation of the matrix. The modification or dissolution properties of the matrix may also occur while re-hydrating the capsules in a final application for example.

The process of the invention makes it possible to obtain particles that have diameters larger that 300 µm, the larger particle size obtainable by prior disclosed spray drying methods, whilst being more cost effective than alternative granule forming types of processes, such as extrusion or coacervation methods, or yet agglomeration techniques.

In other particle forming technologies, such as extrusion technologies with twin screw extruders, the amount of water and the residence times in the extruder are insufficient (in particular for volatile ingredients encapsulation) to permit a proper hydration of any substance capable of reacting with the matrix material and/or proper reaction of the matrix polymer with such a reactant. Moreover, particle diameters between 300 to 800 microns are difficult to obtain. In addition, if any reaction were allowed to occur between a reactant and the matrix, the pressure in the extruder would likely increase to unacceptable values and lead to a de-phasing of the flavour or to expansion of the melt at the die exit.

Other technologies, like vibrating or submerged nozzles, are more expensive than the process of the invention due to a low production capacity for particles below 1000 microns and/or require additional operation units like separation from the bath, washing of the beads and separate drying. Furthermore these operation units are detrimental for example to the flavour or fragrance retention and quality.

Coacervation technologies cannot handle both hydrophilic and hydrophobic actives, nor solid materials. Moreover, coacervation requires an additional drying step that is difficult to achieve and affects the retention of volatiles. Coacervation is also not flexible in terms of choice of the polymer shell material.

Finally, fluidized bed spray granulation or spray agglomeration is also more costly and would not allow the production of similar powders as those of the present invention in a single step process.

### Summary of the Invention

Accordingly, the present invention provides a process for preparing capsules having an average diameter in the range of 100 to 2000 µm and containing an active ingredient, the process comprising the steps of
a) preparing a solution, suspension and/or emulsion comprising the active ingredient, a carrier or matrix material and a solvent to form a feed;
b) spraying the feed in the form of drops having a mean diameter of 100 to 2000 µm into a spraying tower,
c) introducing, simultaneously to the spraying-step, a powdering agent into the spraying tower;
d) supplying, simultaneously to the spraying step, a gas having a temperature in the range of -20°C to 500°C into the spraying tower; and,
e) removing the capsules from the spraying tower; wherein, simultaneously with the addition of the powdering agent used in step c), there is introduced into the spraying tower a substance capable of reacting with said carrier material to modify the dissolution properties of the obtained capsules in aqueous media.

In most preferred embodiments of this process, the solvent is a water-based solvent, and preferably water.

According to particular embodiments of the process of the invention, the latter is a spray-drying process wherein a hot gas is supplied into the spraying tower, at a temperature of 50°C to 500°C, more preferably above 120°C, in step d) of the process. Surprisingly, in spite of the use of such high temperatures, the loss of volatiles encapsulated according to this embodiment was significantly lower than in prior known spray drying processes wherein lower hot air temperatures were employed.

In such spray drying embodiments of the process, the temperatures of the hot air are advantageously in the range of 100°C to 250°C, and most preferably in the range of 120°C to 200°C.

Other aspects of the invention, relate to spray-cooling processes, i.e. processes wherein the air supplied in step d) is at a temperature not above 30°C, more preferably between -20°C and 10°C.

In other particular and preferred embodiments of the process of the invention, the powdering agent comprises the substance capable of reacting with the carrier material of the feed.

Specific embodiments of the process relate to a multistage spray drying or spray cooling process wherein the formed particles are collected in a fluidised bed drier or cooler provided at the bottom of the spraying chamber. Optionally, a second drying or cooling device may be integrated thereafter to allow more precise control of the drying or cooling of the particles.

The present invention also provides particles obtainable by the above-mentioned process and the use of such particles in consumer products traditionally improved or modified by means of flavouring or perfuming ingredients, nutritional supplements such as vitamins or polyunsaturated fatty acids, antioxidant or antibacterial agents, dyes and other substances have a functional purpose.

Preferably, the present invention provides capsules having an average diameter in the range of 100 to 2000 µm encapsulating a flavour and/or fragrance ingredient, the load of the ingredient being in the range of 18-50 wt %, preferably 20-40 wt %, the capsules comprising a coating provided by a powdering agent, whereby this coating provides 0.1 to 30 wt % of the total weight of the capsules.

The present invention further provides a method for controlling the water dissolution properties of sprayed particles containing an active ingredient and having an average diameter in the range of 100 to 2000 µm, wherein the particles are prepared according to the process described above.

In another aspect, the present invention provides the use of a multi stage drying apparatus for preparing capsules according to the method of the present invention.

The process of the present invention entails a number of important advantages over the processes known from the prior art. First of all, it is a one step, low cost process, allowing the production of large particles and tolerating spray drying or cooling of formulations having higher viscosity an a stickier texture than those used in prior art spraying processes. The obtained solids provide a better control of the active ingredient release as they can be designed to have a variety of water solubility behaviours, from being insoluble, to being partially or slowly soluble, or yet characterized by very fast solubility in aqueous media. Moreover, the obtained particles can have dense walls, unlike what is the case of agglomerates. It was also observed that the particles were on average more spherical.

### Description of the figures

Figure 1 shows dry granules obtained according to the invention.
Figure 2 shows the particles of Figure 1 after residence in water for 30 minutes at 50°C.

### Description of Detailed Embodiments of the Invention

The present invention provides a process as recited above for preparing capsules having an average diameter in the range of 100 to 2000 µm encapsulating an active ingredient.

By an "active ingredient" it is meant throughout this description any individual ingredient or mixture of ingredients capable of being encapsulated in a matrix such as described here-below via a spray drying or spray cooling process and providing a functional effect of the flavouring, perfuming, pharmaceutical, nutritional, cosmetic, cleaning, antibacterial or deodorizing, antioxidant or yet softening type, intended for use in a consumer product.

This designation shall include in particular flavours and/or fragrances, meaning ingredients capable of imparting or modifying the odour and/or taste and texture properties of solid or liquid consumer products traditionally perfumed and/or flavoured, such as perfumes, soaps, cosmetics and other body care products, hair care products, foods or beverages, chewing gums or oral care products such as mouthwashes and toothpastes, or yet pharmaceuticals, food supplements and other such products.

The expression "active ingredient" also includes materials other than flavours and fragrances, which can be advantageously encapsulated according to the invention and thus entrapped in a glass matrix. Examples are functional additives such as sugar replacement materials, pharmaceuticals, vitamins and diet or nutritional supplements and therapeutic agents. In particular, ingredients such as polyunsaturated fatty acids, or commercial oils rich in such acids, which will remain stable and retain pleasant taste in the capsules of the invention.

Antioxidants and vitamins, for example, ascorbic acid (vitamin C), tocopherol (vitamin E), or mixtures thereof, are also "active ingredients" in the context of the invention, as are any other common nutritional or cosmaceutical and food ingredients that may generally benefit from encapsulation according to the process of the invention.

Moreover, deodorizing, malodour-counteracting and antibacterial or antimicrobial compounds and compositions, traditionally employed in body or air deodorants or in the preservation of food and cosmetic products, are also "active ingredients" according to the invention.

The "active ingredient" according to the invention further comprises therapeutically active and pharmaceutical ingredients.

The process of the present invention may be performed in any conventional spraying tower such as described for example in WO 2004/062382 (in particular on page 2, lines 26-35). A conventional multi-stage drying apparatus is quite appropriate for conducting the steps of this process. It comprises a spraying tower, and, at the bottom of the tower, a fluidised bed intercepting partially dried particles after falling across the tower. In a typical multi-stage drier, an air inlet and an air outlet are located in the upper region of the tower. Generally, it is possible to maintain, in these devices, a suspension or cloud of fine particles, herein referred to as a "powdering agent", as is required for the purpose of the present invention. The powdering agent is generally introduced by a separate inlet that may preferably be situated either close to the inlet of the aqueous emulsion or in the lower part of the drying tower. The powdering agent is, in the operation mode, in constant movement and may exit the tower together with the air outlet to be returned back into the same tower.

The capsules produced according to the present invention have an average diameter in the range of 100, and more preferably 200, to 2000 µm. Preferably, the average diameter is in the range of 200 to 800 µm, more preferably from 300 to 500 µm. The term "average" and/or "mean" as used for example in the expression "average diameter" refers to the arithmetic mean.

The size of the capsules is determined by the size of the drops that are dispersed into the drying tower according to a step of the method of the invention. According to an embodiment of the invention, the starting emulsion or suspension of active ingredient and carrier or matrix material, i.e. the feed, is guided through a spraying nozzle or through a centrifugal wheel disk into the spraying tower. Other devices may be used to disperse the feed into the form of droplets of a controlled average size. For instance, vibrating orifices may be used instead.

If a spraying nozzle is used for dispersing the drops, their size may be controlled by the flow rate of an atomising gas introduced through the nozzle, for example. When a centrifugal wheel disk is used for dispersal, the main factor for adjusting droplet size to the terms of the present invention is the centrifugal force with which the drops are dispersed from the disk into the tower. The centrifugal force, in turn, depends on the speed of rotation and the diameter of the disk. The feed flow rate, its viscosity and surface tension, as well as the disk geometry, are also parameters controlling the final drop size and size distribution. By adjusting these parameters, the skilled person can control the size of the drops of the composition to be dispersed in the tower.

The process of the present invention preferably comprises the step of preparing an aqueous solution, suspension and/or emulsion, commonly designated as the feed, comprising water, the active ingredient, the matrix materials and possibly an additional reactant capable of modifying such matrix material by reacting with the reactant present in the powdering agent.

Within the context of this specification the word "comprises" is taken to mean "includes, among other things". It is not to be construed as meaning "consists only of".

The active ingredient, typically a flavour and/or fragrance, may thus be mixed with a carrier system by dissolving it together with a polymer or other matrix material, by suspending and/or by emulsifying it therein. For the sake of convenience, the term emulsifying is used to encompass all the different forms of mixing the active ingredient with the matrix materials.

The active ingredient may be hydrophilic and/or hydrophobic. Accordingly, the invention is applicable to a large family of ingredients including water soluble ones, such as juices or reaction flavours, for example. The ingredient may also comprise a combination of hydrophilic and hydrophobic components. If the ingredient is hydrophilic, it will generally be dissolved in the aqueous solution together with the matrix materials. According to a preferred embodiment, the ingredient comprises hydrophobic components, while the matrix materials may be soluble in water. Generally, in this case, the hydrophobic ingredient is emulsified in the form of small droplets having an average diameter in the range of 0.5-10 µm, preferably 1-5 µm for example, within the matrix materials. Emulsification may be made, for example, by using a high pressure homogeniser or a colloid mill, preferably in the presence of an emulsifier

Flavour and/or fragrance active ingredients or compositions encompass ingredients of current use in the flavour and/or fragrance industry, of both natural and synthetic origin. It includes single compounds and mixtures. The capsules used in the invention can encapsulate volatile or labile ingredients in liquid form, preferably with a log P in the range of -2 and 7, preferably 2 - 6. Specific examples of such components may be found in the current literature, e.g. in Fenaroli's Handbook of flavour ingredients, 1975, CRC Press; Synthetic Food adjuncts, 1947 by M.B. Jacobs, edited by Van Nostrand; or Perfume and Flavour Chemicals by S. Arctander, 1969, Montclair, New Jersey (USA).

Accordingly, in a preferred embodiment, the ingredient is selected from the group consisting of a flavour and/or fragrance compound or composition, a flavour or fragrance natural extract, and a mixture of any of the before mentioned ingredients.

Flavour and/or fragrance ingredients are well known to a person skilled in the art of aromatising and/or providing a pleasant odour to consumer products, i.e. of imparting an odour or a flavour or taste to a consumer product, or of modifying the taste and/or odour of said consumer product. Natural extracts can also be encapsulated into the system for flavouring or perfuming consumer end products. Examples of these include, amongst others, citrus extracts such as lemon, orange, lime, grapefruit, or mandarin oils or coffee, tea, mint, cocoa or essential oils of herbs and spices.

Other examples of flavour and/or fragrance active ingredients are synthetic flavour and/or fragrance oils, flavouring aromatics and natural extracts such as oils, essential oils, oleoresins and other extracts derived from plants, for example from leaves, flowers, fruits, roots, rhizomes, stem, and so forth.

Flavours included by the term flavour ingredient are reaction flavours, or processed flavours. These flavours may be obtained by heating food ingredients and/or ingredients appropriate for use in foodstuffs or in processed flavourings. A nitrogen source and a carbohydrate source are necessary to perform a Maillard type reaction for obtaining such processed flavours.

The terms flavour and fragrance also include compounds such as cooling, refreshing, salivating, pungent, tingling and hot/spicy compounds of current use in fragrances and flavours.

The flavour and/or fragrance ingredient may be present in the form of a mixture with solvents, adjuvants, additives and/or other components, generally those of current use in the fragrance and/or flavour industry.

A typical characteristic of flavour and/or fragrance ingredients is the high proportion of volatile compounds and/or components. Accordingly, in an embodiment, the ingredient comprises at least 10 wt %, preferably at least 20 wt %, more preferably at least 30 wt % and most preferably at least 40 wt % of chemical compounds having a vapour pressure of ≥ 0.007 Pa at 25°C. Preferably, at least 10 wt % have a vapour pressure of ≥ 0.1, more preferably, at least 10 wt % have a vapour pressure of ≥ 1 Pa at 25°C, and most preferably, at least 10 wt % have a vapour pressure of ≥ 10 Pa at 25°C. The value of 0.007 Pa at 25°C is selected because it encompasses most of the compounds used by the skilled flavourist and/or perfumer. Compounds meeting these criteria are generally regarded as having a volatile character. In addition, compounds that remain odourless due to a lower volatility are excluded. The limit of 10 wt % of such highly volatile compounds is regarded as being a substantial part of the active flavour or fragrance ingredient that can be encapsulated according to prior known spray drying processes.

For the purpose of the present invention and for the sake of convenience, the vapour pressure value is determined by calculation. Accordingly, the method disclosed in "EPI suite"; 2000 U.S. Environmental Protection Agency, is used to determine the concrete value of the vapour pressure of a specific compound or component of the active ingredient.

In the context of the present invention, percentages are percentages by weight of dry matter, unless otherwise indicated. Similarly, if proportions are indicated as parts, parts of weight of dry matter are meant. Where water is indicated as part of a composition, percentages refer to the total weight of the composition, including water.

Matrix materials useful in the preparation of the feeds in a step of the present invention have the purpose of providing a carrier for the active ingredient. Preferably, the matrix materials are selected on the basis of their capacity to form a film or a glassy matrix upon the drying of the feed dispersed drops. A glassy matrix is an amorphous solid characterized by viscosities of the order of about 10¹⁰ to 10¹² Pa*s and an extremely low molecular mobility. A good understanding of the glassy state is provided by Dominique Champion et al in "Towards an improved understanding of glass transition and relaxations in foods: molecular mobility in the glass transition range", Trends in Food Science and Technology 11 (2000) 41-55.

Matrix components may be selected, for example, from polymers, namely proteins and pectin in particular, a very convenient material in food supplement and nutraceutical products, polymeric carbohydrates, and other polymeric materials. The polymeric materials preferably comprise hydrophilic polymers in order to provide an effective oxygen-barrier. Accordingly, the matrix may comprise hydrocolloids. In addition, less water soluble polymers, i.e. hydrophobic polymers, may also be present in the matrix in order to provide some lipophilic character to the glassy matrix and thus to provide protection against moisture. In addition, the matrix may contain further components that are not polymeric, but that may assist in the formation of a dense glassy matrix or that may be added for other purposes.

Suitable proteins include caseins, whey proteins, soy protein, pectin and/or gelatine, for example. These proteins have good emulsification and film forming properties and can thus form the basis for polymer matrices.

The matrix component may comprise carbohydrates.

For example the matrix component may comprise monosaccharides, disaccharides, trisaccharides, oligosaccharides, polysaccharides or mixtures thereof.

Suitable monosaccharides include D-Apiose, L-Arabinose, 2-Deoxy-D-ribose, D-Lyxose, 2-O-Methyl-D-xylose, D-Ribose, D-Xylose, which are all Pentoses or Hexoses like for instance L-Fucose , L-galactose, D-Galactose, D-Glucose, D-Mannose, L-Rhamnose, L-mannose, or mixtures of several of these.

Mono- and dissacharides may be reduced to the corresponding alcohols, for example xylitol, sorbitol, D-mannitol and/or maltitol. Similarly, oxidation to aldonic, dicaroxyclic acids or uronic acids and reactions with acids, alkalis or amino compounds can give rise to many other compounds like isomaltol, for instance, which may be comprised in the matrix component of the present invention.

Suitable oligosaccharides are molecules consisting of from 3-10 monosaccharide units, such as maltopentaose, fructo- and/or galactooligosaccharides.

Polysaccharides, that is, saccharides containing more than 10 monosaccharide units per molecule are particularly preferred. These polymers can be either perfectly linear (cellulose, amylose), branched (amylopectin, glycogen) or linearly branched. They can include carboxyl groups (pectin, alginate, carboxymethyl cellulose) or strongly acidic groups (furcellaran, carrageenan or modified starch). They can be modified chemically by derivatization with neutral substituents (in the case of methyl ethyl cellulose or hydroxypropyl cellulose for instance) or acidic substituents (with carboxymethyl, sulphate or phosphate groups).

The matrix component may comprise gums and/or hydrocolloids, for example, gum Arabic, agar agar and the like.

Preferably, the matrix component comprises a starch and/or a starch derivative such as pre-gelatinised starch, thin- or thick-boiling starch, dextrins or maltodextrins of various molecular weights. Most preferably, the matrix component comprises maltodextrin and/or corn starch syrup having a mean dextrose equivalence of 5-25, preferably 6-20, more preferably 10-18. Other possible modifications of starch and resulting derivatives suitable as a matrix component include octenyl-succinated starch, starch ethers (e.g. carboxymethyl starch), starch esters (e.g. starch monophosphate), cross-linked starch and/or oxidised starch.

In a preferred embodiment, the matrix component comprises, per dry weight of the glassy matrix alone, 60-95 wt % of maltodextrin, preferably with a DE value in the above indicated ranges, and 5-40 wt % of modified starch, such as alkenyl-succinated starch, (in particular octenyl-succinated starch).

While the above matrix-materials are preferred in case of applications of the capsules to food products and/or beverages, there may be numerous further materials suitable when the capsules are not intended for addition to food products. For example, in capsules comprising fragrance or malodour control ingredients, the matrix material may be selected from a much wider range of materials. These include synthetic polymers, copolymers or natural non-food polymers, salts and other fillers may be used as matrix materials. These include cellulose and/or hemi-cellulose, as well as cellulose derivatives.

The matrix component may comprise mixtures of the above- and/or below mentioned carbohydrates, their derivatives and/or proteins. For example, mono-di or trisaccharides and/or their reaction products (see above) may be used as additives in combination with a protein or polysaccharide based matrix and thus bring properties as desired to the matrix component.

The feed prepared in a step of the present method preferably comprises 10-70 wt % of matrix materials, 30-70 wt % of water and 5-35 wt % of active ingredient. More preferably, the percentages are in the range of 20-40, 40-60 and 10-30 wt %, and most preferably 25-35, 45-55 and 15-20 wt % of matrix materials, water and active ingredient, respectively, in the spray drying feed.

According to a preferred embodiment of the process of the present invention, the feed comprising the active ingredient, matrix components and water has a dry matter content in the range of 45-80 wt %, preferably 50-70 wt %. The relatively high dry-matter content entails a high viscosity emulsion, rather difficult to handle and guide into the spraying tower. On the other hand, thanks to a high dry-matter content, the drying process becomes more efficient as less water needs to be evaporated. Unlike in conventional spray drying, where the feed's droplet diameter size is less than 150 µm, the present invention enables a successful drying of comparatively larger drops. Feed of higher viscosity can be dried because it is not necessary to create a fine spray, the use of the powdering agent making it possible to dry very large drops.

The present invention comprises a step of introducing, simultaneously to the spraying-step, a powdering agent into the spraying tower. Such powdering agents are mainly intended to provide a coating of powdering agent on the drops of the sprayed feed. According to the invention, this powdering agent may comprise, in addition to the coating or anti-caking component, a component capable of reacting with the material or materials of the encapsulating matrix to modify the dissolution properties of the capsules in aqueous media.

The "coating or anti-caking component" forming part or the whole of the powdering agent according to the invention is intended to mean here any ingredient that is capable of sticking to the wet feed droplet and absorb water to a certain degree. Such agents are preferably selected from the group consisting of starch, starch derivatives, talc, bentonite, silicon dioxide, calcium, magnesium, aluminium or potassium silicates, sodium, potassium or calcium aluminosilicates, calcium, sodium or magnesium carbonates, calcium or magnesium phosphates, ferric ammonium citrate, microcrystalline cellulose and cellulose derivatives or fibres, aluminium, calcium, sodium, magnesium, potassium or ammonium salts of fatty acids, magnesium oxides and mixtures comprising two or more of the aforementioned powdering agents. The purpose of this coating or anti-caking component is to prevent the dispersed feed drops from attaching to each other and/or to the inner walls of the spraying tower. It may be present in a proportion of 0.1 to 30% by weight of the feed's total solids weight.

The "reacting component or reactant" agent, possibly a part of the powdering agent, is intended to mean here any compound or substance that is capable of reacting with the carrier or matrix materials of the feed being sprayed in order to induce gelling, polymerisation, cross linking, crystallization or any other chemical or physical transformation of said matrix material susceptible of modifying its water dissolution properties. Thus the nature of the reacting component depends on the nature of the matrix material used in the feed of active ingredient and can therefore be selected by the person skilled in the art accordingly.

For inducing the modification of the dissolution properties of the matrix, it is essential to have a reactant in the feed that can react with at least one of the feed's components, either during the spraying of the feed or at a later stage when the solid capsules are incorporated into an end product where this reaction becomes possible. Thus at least two components must be present in the spraying tower, one gelling agent, monomer or polymer in the feed and a reactant, preferably a solid material, capable of reacting with the gelling agent, monomer or polymer of the feed to modify the water solubility of the final matrix encapsulating the active ingredient in the obtained granules.

According to particular embodiments of the invention the reactant or reacting component is part of the powdering agent and is a solid material admixed with the coating or anti-caking component, both being fed through the same inlet into the spraying tower as the feed is being sprayed. The particles of the feed will thus be coated with the anti-caking agent whilst their matrix undergoes the modifying reaction capable of changing its water dissolution properties.

The powdering agent is thus suspended in the tower and creates a coating on the dispersed drops as soon as they enter the tower. Accordingly, in a preferred embodiment of the invention, particles of the powdering agent are suspended in the spraying tower and circulate from an inlet into the spraying tower to an outlet, which outlet is also the outlet of the hot gas.

Generally, the powdering agent itself moves in circuit from an inlet for the powdering agent into the drying tower, to an outlet where it exits together with exhaust gas. The powdering agent may be recycled in a cyclone collector from which it may be redirected by a powdering agent return pipe to the inlet into the spraying tower. Preferably, the powdering agent circulates, at least partly, in counter current with the dispersed drops of the feed. Accordingly, in a preferred embodiment of the invention, spare powdering agent not absorbed on the dispersed capsules exits the spraying tower together with the hot gas through an outlet of exhaust gas, which leads to a cyclone or a filter bag house separating the hot gas from the powdering agent, and whereby the powdering agent is reintroduced into the spraying tower thus forming a continuous circulation of powdering agent from the cyclone to the spraying tower and back to the cyclone.

The dry anti-caking agent together with the dry reactant forming preferred powdering agents according to the invention will stick onto the wet surface of the large droplets of the feed. The reaction between the reactant and the matrix material can either start directly during the flight of the droplets or be induced by the release of the reactant in application, when the capsules are re-hydrated and/or when they are introduced in a medium in which the pH and/or temperature conditions, for instance, induce the release of the reactant.

The layer of anti-caking agent and possibly the reactant itself will prevent the large droplets from sticking onto the wall of the spraying chamber.

Both the anti-caking and reactant ingredients are preferably formed of powder particles of a diameter size below 100 µm and more preferably 50 µm.

The materials for the reactant or reacting component present in the powdering agent and/or in the feed must be adapted to the nature of the matrix material.

It goes without saying that this reactant may also be introduced in the spraying tower separately from the anti-caking component of the powdering agent. They must however be present simultaneously with the spraying of the feed. Moreover, the reactant may be sprayed in liquid form into the chamber via a second nozzle instead of being mixed dry with the anti-caking component of the powdering agent.

Several polymers/ monomers, gelling agent and/or several reactants can be used.

The reactant can also be introduced in the matrix formula together with the encapsulating polymer, provided that the pH conditions of the feed prevent the reactant from reacting with the polymer. An additional third substance may thus be needed in the tower to modify the spraying medium and thus trigger the reaction between the reactant and the polymer. For example, an insoluble form of a calcium salt may be incorporated in the matrix together with an alginate and the reaction between alginate and calcium triggered by a change in pH induced by the presence of an acid in the end application consumer product or at the surface of the granule upon contact of the latter with water.

The aqueous medium of the feed may contain organic solvents or alternatively the feed medium may be based on such solvents.

The process of the invention may be carried out in any spraying equipment, including any standard type spraying or drop producing device like rotary atomizers, high pressure nozzles, vibrating nozzles etc, a spraying chamber or tower, and a fluidised bed integrated at the bottom of the chamber or separate from it.

Optionally, an additional dryer can be used, similar to those known in the dairy industry equipments used in the 3-stage drying of instant powders.

Dosing units for the continuous introduction of the required amounts of anti-caking agent and reactant may also be provided in the equipment or apparatus of the invention.

The feed must be composed of at least a polymer or film-forming substance or any other ingredient capable of forming an encapsulating matrix to carry the active ingredient. The selection of this polymer may determine the nature of the reactant forming part of the spraying atmosphere and preferably incorporated in the powdering agent or alternatively, if an encapsulating polymer is inadequate to react with a particular reactant, a second matrix material may be added, namely a second polymer, gelling agent or monomer capable of reacting with the reactant to modify the matrix properties and dissolution properties in an aqueous medium and hence the kinetics of release of the active ingredient.

The process of the invention may be a spray drying or spray cooling process.

The so-called spray cooling differs from standard spray drying in the sense that the feed already contains a gelling agent or a compound having a melting point and capable of hardening the encapsulating matrix on cooling.

The same constraints as those of current spray drying processes are associated with spray cooling since the feed must also have a low viscosity and only fine droplets of less than 300 microns diameter can be properly solidified on cooling. The feed is always sticky so that the geometry of the current spraying chambers must be very large to permit a proper cooling of such large particles.

According to the invention, the spray cooling process involves the spraying of a feed comprising a matrix or carrier material capable of hardening on cooling either by gelling, crystallizing or solidifying. The feed is atomized in large droplets and comes into contact in the tower with the dry powdering agent containing the reactant capable of modifying the dissolution properties of this matrix.

Both spray drying and spray cooling processes of the invention present the advantage of permitting the use of very sticky feed formulations, whereas in standard spraying processes the feed formula must be adapted to avoid the product to stick everywhere in the spraying chamber. Thus, standard feed formula could not contain large amounts of sugars or plasticizers for instance. The present invention solves this problem since the feed can contain raw materials that are difficult to handle by standard spray drying or spray cooling techniques.

The formed granules are collected in a fluidised bed at the bottom of the chamber where the chemical or physical reaction can eventually continue further until complete drying or hardening of the granules is achieved.

The inlet temperature of the air in the spraying tower may vary in a wide range of values, comprised between -20°C and +500°C. The process can be carried out either using a hot gas in order to dry the particles at least partially during their flight, or a cold gas to induce the hardening of the droplets, depending on the nature of the feed. As previously mentioned, particular embodiments of the process relate to inlet temperatures above 120°C.

In the case of a high inlet temperature (e.g. 150°C) a partial drying of the granules takes place in flight and the drying continues in the fluidized bed until the granules have reached the required final residual moisture.

In the case of low inlet temperatures (e.g. 5°C), the feed must either contain a gelling agent capable of hardening the matrix on cooling, or the chemical reaction between the feed and the reactant must be fast enough to sufficiently harden the droplets and adequately collect them in the fluidized bed. The granules are then dried or further hardened in a second phase of the process where the temperature of the fluidized bed is increased or in a separated part of the equipment (additional external fluid bed dryer/cooler for instance).

As previously mentioned, there is supplied into the spraying tower a hot gas having a temperature in the range of 121-250°C. In such embodiments of the invention the hot gas is supplied at a higher temperature than those disclosed in the prior art. It has been, surprisingly established that relatively large-sized drops comprising the active ingredient are preferably dried under supply of a gas having a higher temperature while having less loss during drying. Without wishing to be bound by theory it is hypothesized that the higher temperature in the spraying zone brought about by the higher temperature of the inlet gas stream results in a shorter drying time. According to the invention, more water is evaporated from the drop during its flight, and the subsequent drying time in the fluidised bed is also shorter.

According to even more preferred embodiments, the hot gas supplied into the drying tower has a temperature in the range of 130-220°C, preferably 140-210°C, and most preferably 150-190°C.

The supply of hot gas through a hot gas inlet results in a temperature in the spraying tower in the range of about 180 to about 50°C, preferably about 170°C to about 40°C. The temperature in the tower follows a gradient, with the highest temperature at the hot gas inlet, which thus provides a high temperature at the spraying zone. At the bottom of the tower the temperature is much lower, due to the evaporation of water from the dispersed drops.

The method of the invention comprises the step of removing capsules comprising a coating of powdering agent from the spraying tower.

The process can be performed in batch or continuous mode. Accordingly, in batch mode, there will be provided an outlet at the level of the fluidised bed leading to a collecting vessel. By turning off the fluidised bed and opening the valve leading to the collecting vessel, the capsules are guided, for example by gravity, to the collecting vessel, from where they may be removed. In a preferred embodiment, the method of the invention is performed continuously, for example by conveying the granules from the integrated fluidised bed to an external, additional fluidised bed where the capsules may be further dried to accurately control their final moisture content. In the continuous mode, the residence time of the capsules in the whole process is reduced which is found to be beneficial to the quality of the product.

Preferably, the fluidised bed is supplied with a gas having a temperature in the range of 20°C to about 100°C, meaning that gas having a temperature in this range is supplied from below the bed through small apertures to fluidise/intercept the capsules. Preferably, the temperature of the gas is in the range of 65 to 90°C. Generally, the gas is air. Similarly to the spraying zone, the method of the present invention again provides for comparatively high temperatures in the fluidised bed in view of the prior art. Again a shorter drying period results in surprisingly lower loss of volatile ingredients.

The capsules obtainable and/or obtained by the method of the present invention may have an active ingredient load, e.g. a flavour and/or fragrance load, of 1-50 wt %. According to a preferred embodiment, the load of the active ingredient is in the range of 18-50 wt %. It is thus possible to have high active ingredient loads in the range of 20-40 wt %, preferably 25-38 wt % and more preferably 27 to 35 wt %.

Higher ingredient loads are obtainable by the processes of the present invention wherein comparatively high temperatures of the inlet gas are used, thus resulting in distinctly superior volatile ingredient loads being obtained than when prior known methods are used. The high loads are further a consequence of reduced loss of volatiles achieved by the present method.

Generally, the contribution of the powdering agent to the total weight of the capsules may be in the range of 0.1 to 30%, which corresponds to a preferred embodiment of the present invention. However, the present invention allows for the reduction of the amount of powdering agent to 0.1 to 27 wt %, preferably 0.5 to 25 wt %, more preferably 1 to 20 wt % and most preferably 2-15 wt % of powdering agent. In a preferred embodiment, the amount of powdering agent makes up ≤ 10 wt % of the total weight of the capsules of the present invention.

The present invention further provides capsules obtainable and/or obtained by the method of the present invention.

The present invention also provides a food product comprising the capsules of the invention and/or the capsules obtainable by the method of the invention. Products may be, for example, food products in which the active ingredient of the capsules is a flavour ingredient.

The invention also encompasses perfumed products comprising the capsules of the invention, in which the active ingredient is a perfuming composition, for example a perfume, an eau-de-toilette, or another composition of perfuming ingredients optionally further comprising solvents and/or other adjuvants.

The encapsulation of active pharmaceutical ingredients is also encompassed by the present invention which thus also relates to medicinal formulations and pharmaceutical products containing them.

The application of the capsules obtainable by the process of the invention is most useful and advantageous whenever controlled release of active ingredients is desired upon use in an aqueous or moisture containing environment. The preparation of powders for instant drinks for example will especially benefit from these capsules as will instant soup preparations, taste-masking applications, chewing gums, low fat food or other food applications. These capsules may also find applications in the pharmaceutical or cosmetic industry, when controlled release of an active is desired.

The invention will now be illustrated by way of the following examples.

### Examples

In the following examples the following general conditions were applied: a small scale spray drier/cooler of the Niro^{®} FSD0.8 type, with integrated fluidized bed was used to carry out the processes; in this spray drier, the main air stream was introduced in an annular space around the atomizing nozzle on top of the spraying chamber and also exhausted also from the ceiling of the same chamber; the exhaust air passed through a cyclone where the fine particles, including the anti-caking agent and the reactant, were collected and returned into the chamber using an additional air stream; a fluidized bed was integrated at the bottom of the chamber to collect the wet granules surrounded by the anti-caking agent and reactant. A third air stream passed through the perforated plate in the fluidized bed in order to dry the granules.

### Example 1

### Preparation of capsules containing limonene

### a) Preparation of the dry anti-caking agent and reactant mixture forming the powdering agent

250 G of native Starch type B from Roquette Frères SA were mixed with 150 g of dry Calcium Gluconate (Fluka) using a mechanical blender (HUG Maschinenbau, Switzerland). 0.4 G of fumed silica, Aerosil^{®} 200 from Degussa were added to increase the flowability of the powder mixture.

### b) Preparation of the feed

65.5 G of modified starch (Capsul^{®}, National Starch), 5 g of citric acid and 50 g sucrose were dissolved together into 1150 g of warm water (50°C) using a dissolver disk until a clear yellow solution was obtained. 29.5 G of alginate (protanal LF10/60 from FMC Biopolymer) was then added until complete dissolution. 150 G of pure limonene were emulsified into the modified starch solution using an UltraTurrax^{®} UT25 rotor/stator high shear mixer rotating at 23000 rpm for 2 minutes.

### c) Processing conditions

- Inlet main air stream temperature = 100°C
- Inlet main air stream flow rate = 55 Kg/h
- Fluidized bed air stream temperature = 65°C
- Fluidized bed air stream flow rate = 24 Kg/h
- Fine return air stream temperature = 50°C
- Fine return air stream flow rate = 11 Kg/h
- Nozzle gas flow rate = 3 Kg/h
- Diameter of the nozzle = 1mm
- Feed temperature = 40°C

At the beginning of the process the powdering agent mixture (i.e. the native starch and Calcium gluconate blend) was introduced in the chamber via a hole in the ceiling. This powder mixture was thus immediately fluidized into the chamber and recycled in closed loop continuously.

After all the temperature settings had reached the desired values in the apparatus, the feed was atomized during 10 minutes at a feed flow rate of 0.055 l/min.

The atomization was then stopped and the granules were kept fluidized into the fluidized bed during 15 more minutes.

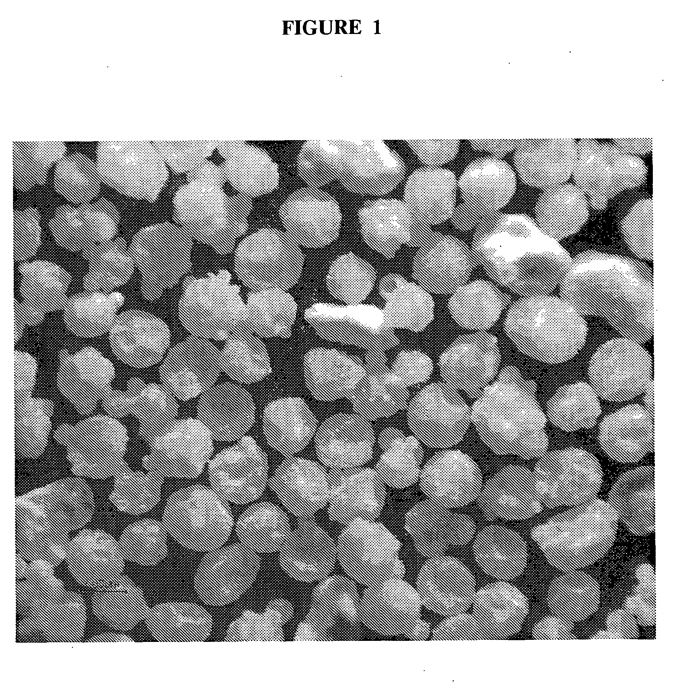

180 G of dry granules were collected in a glass vessel connected to the fluidized bed via a butterfly valve. Their average diameter was of 350 microns. Figure 1 shows the general aspect of the obtained dry granules.

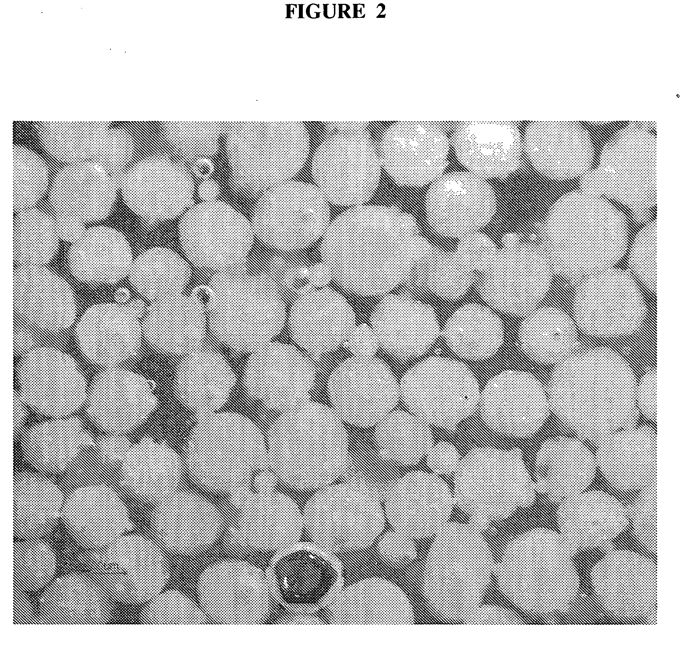

In order to check that the granules were insoluble in water, 1 g of these granules were introduced into 500 ml of cold water. After hydration white granules were clearly visible and the water phase remained clear, as is shown in Figure 2. The limonene emulsion droplets were trapped into an alginate gel and couldn't migrate into the water phase.

### Example 2

### Preparation of capsules containing limonene

### a) Preparation of the dry anti-caking agent and reactant

200 G of native Starch type B from Roquette Frères SA were mixed with 100 g of dry Calcium Gluconate (Aldrich 22,764-1) using a mechanical blender (HUG Maschinenbau, Switzerland).

### b) Preparation of the feed

100 G of modified starch (Capsul^{®}, National Starch), 640 g of maltodextrin 10-18DE and 60 g of pectin (Unipectine OF 305 from Degussa) were dissolved together into 1100 g of warm water (50°C) using a dissolver disk until a clear yellow solution was obtained. 200 G of pure limonene were emulsified into the modified starch solution using an UltraTurrax^{®} UT25 rotor/stator high shear mixer rotating at 23000 rpm for 2 minutes.

### c) Processing conditions

- Inlet main air stream temperature = 210°C
- Inlet main air stream flow rate = 75 Kg/h
- Fluidized bed air stream temperature = 75°C
- Fluidized bed air stream flow rate = 24 Kg/h
- Fine return air stream temperature = 50°C
- Fine return air stream flow rate = 11 Kg/h
- Nozzle gas flow rate = 20 nl/min
- Diameter of the nozzle = 1mm
- Feed temperature = 40°C

At the beginning of the process the powder mixture was introduced into the chamber via a hopper connected to the fine return pipe. This powder mixture was thus immediately fluidized into the chamber and recycled in closed loop continuously.

After all the temperature settings were obtained in the equipment, the feed was atomized during 10 minutes at a feed flow rate of 0.057 l/min.

The atomization was then stopped and the granules were kept fluidized into the fluidized bed during 10 more minutes.

270 G of dry granules were collected in a glass vessel connected to the fluidized bed via a butterfly valve. Their average diameter was of 500 microns.

In order to check that the granules were insoluble in water, 1 g of these granules were introduced into 1000 ml of warm water (60°C). After hydration white granules were clearly visible and the water phase remained clear. The limonene emulsion droplets were trapped into a pectin gel and couldn't migrate into the water phase.

The final limonene load was of 15% w/w based on the final weight of the granules. The granule contained on their surface about 20% w/w of a mixture of dry Ca-gluconate and native starch. The loss of flavour compared to the amount contained in the original feed was less than 10%, showing that the process is very effective for the encapsulation of such highly volatile active ingredients. Note that the final amount of Ca-gluconate and native starch present at the surface of the granules has to be taken into consideration for the calculation of the flavour losses.

### Example 3

### Preparation of capsules containing limonene

The same processing conditions as those of Example 2 were used, except that the formulation contained a lesser amount of pectin.

### Preparation of the feed:

100 G of modified starch (Capsul^{®}, National Starch), 680 g of maltodextrin 10-18DE and 20 g of pectin (Unipectine OF 305 from Degussa) were dissolved together into 1100 g of warm water (50°C) using a dissolver disk until a clear yellow solution was obtained.

200 G of pure limonene were emulsified into the modified starch solution using a rotor/stator high shear mixer UltraTurrax^{®} UT25 rotating at 23000 rpm for 2 minutes. 270 G of dry granules were collected in a glass vessel.

The final limonene load was also of 15% w/w based on the final weight of granules. Although the amount of pectin was much lower, the particles still jellified in warm water and the limonene droplets were trapped in a gelled matrix.

### Examples 4 and 5

### Preparation of flavour-containing capsules

Two flavours, a minty tonality flavour (880335 NF from Firmenich SA), respectively a strawberry tonality flavour (502301 TA from Firmenich SA), were encapsulated in identical conditions to those described in Example 3 to provide flavoured capsules of flavour active material and which behaved similarly when introduced in warm water. The final flavour load was 33.3 weight % for the minty flavoured capsules, respectively 35.2 weight % for the strawberry flavoured capsules, in both cases the particles having an average diameter of 350 micron.

### Example 6

### Preparation of capsules containing perfume

### a) Preparation of the dry anti-caking agent and reactant mixture forming the powdering agent

225 G of native Starch type B from Roquette Frères SA were mixed with 75 g of dry DiSodium Sulfate (Na₂SO₄ - Fluka) using a mechanical blender (HUG Maschinenbau, Switzerland).

### b) Preparation of the feed

207.6 g of Polyvinyl Alcohol (Erkol V03/1410), 28 g of Sucrose and 0.4 g of antifoam, were dissolved together into 800 g of warm water (50°C) using a dissolver disk until a clear yellow solution was obtained. 164 g of Perfume (Loving UN 147988) were emulsified into the Polyvinyl Alcohol/sucrose solution using an UltraTurrax^{®} UT25 rotor/stator high shear mixer rotating at 23000 rpm for 2 minutes.

### c) Processing conditions

- Inlet main air stream temperature = 230°C
- Inlet main air stream flow rate = 75 Kg/h
- Fluidized bed air stream temperature = 75°C
- Fluidized bed air stream flow rate = 23 Kg/h
- Fine return air stream temperature = 50°C
- Fine return air stream flow rate = 16 Kg/h
- Nozzle gas flow rate = 3 Kg/h
- Diameter of the nozzle = 1 mm
- Feed temperature = 40°C

At the beginning of the process the powdering agent mixture was introduced in the chamber via a hopper connected to the fine return line. This powder mixture was thus immediately fluidized into the chamber and recycled in closed loop continuously.

After all the temperature settings had reached the desired values in the apparatus, the feed was atomized during 10 minutes at a feed flow rate of 0.055 l/min.

The atomization was then stopped and the granules were kept fluidized into the fluidized bed during 15 more minutes.

180 G of dry granules were collected in a glass vessel connected to the fluidized bed via a butterfly valve. Their average diameter was of 350 microns.

In order to check that the granules were insoluble in water, 1 g of these granules were introduced into 500 ml of cold water. After hydration white granules were clearly visible and the water phase remained clear, as is shown in Figure 2. The perfume emulsion droplets were trapped into a Polyvinyl alcohol matrix which dissolved very slowly since Polyvinyl Alcohol precipitate in presence of the Disodium Sulfate.

### Example 7

### Preparation of capsules containing limonene by internal gelation

### a) Preparation of the dry anti-caking agent and reactant mixture forming the powdering agent

250 G of native Starch type B from Roquette Frères SA were mixed with 150 g of dry Citric acid using a mechanical blender (HUG Maschinenbau, Switzerland). 0.4 G of fumed silica, Aerosil^{®} 200 from Degussa were added to increase the flowability of the powder mixture.

### b) Preparation of the feed

60.5 G of modified starch (Capsul^{®}, National Starch) and 50 g sucrose were dissolved together into 1150 g of warm water (50°C) using a dissolver disk until a clear yellow solution was obtained. 10 G of Dicalcium Phosphate (Fluka) were added to the solution and a suspension of Dicalcium phosphate in the modified starch/sucrose solution was created. 29.5 G of alginate (protanal LF10/60 from FMC Biopolymer) was then added until complete dissolution. 150 G of pure limonene were emulsified into the suspension of Dicalcium Phosphate solution using an UltraTurrax^{®} UT25 rotor/stator high shear mixer rotating at 23000 rpm for 2 minutes.

### c) Processing conditions

- Inlet main air stream temperature = 100°C
- Inlet main air stream flow rate = 55 Kg/h
- Fluidized bed air stream temperature = 65°C
- Fluidized bed air stream flow rate = 24 Kg/h
- Fine return air stream temperature = 50°C
- Fine return air stream flow rate = 11 Kg/h
- Nozzle gas flow rate = 3 Kg/h
- Diameter of the nozzle = 1mm
- Feed temperature = 40°C

At the beginning of the process the powdering agent mixture (i.e. the native starch and citric acid blend) was introduced in the chamber via a hole in the ceiling. This powder mixture was thus immediately fluidized into the chamber and recycled in closed loop continuously.

After all the temperature settings had reached the desired values in the apparatus, the feed was atomized during 10 minutes at a feed flow rate of 0.055 l/min.

The atomization was then stopped and the granules were kept fluidized into the fluidized bed during 15 more minutes.

180 G of dry granules were collected in a glass vessel connected to the fluidized bed via a butterfly valve. Their average diameter was of 350 microns. Figure 1 shows the general aspect of the obtained dry granules.

In order to check that the granules were insoluble in water, 1 g of these granules were introduced into 500 ml of cold water. After hydration white granules were clearly visible and the water phase remained clear. The limonene emulsion droplets were trapped into an alginate gel and couldn't migrate into the water phase.

The Dicalcium Phosphate is an insoluble calcium salt at neutral pH. A gel of alginate was thus formed into the matrix when the citric acid present at the surface of the granules was dissolved in water. The local pH around the granules was decreased and calcium ions were released by the dissolution of Dicalcium Phosphate at such low pH. This principle is called internal gelation of alginate.

### Example 8

### Preparation of capsules containing a sweetener

### a) Preparation of the dry anti-caking agent and reactant

200 G of native Starch type B (Roquette Frères SA) were mixed with 100 g of dry

Calcium Gluconate (Aldrich 22,764-1) using a mechanical blender (HUG Maschinenbau, Switzerland).

### b) Preparation of the feed

100 G of modified starch (Capsul®, National Starch), 660 g of maltodextrine 10-18DE and 40 g of pectin (Unipectine OF 305 from Degussa) were dissolved together into 1100 g of warm water (50°C) using a dissolver disk until a clear yellow solution was obtained. 200 G of aspartame were added to the modified starch solution using an UltraTurrax® UT25 rotor/stator high shear mixer rotating at 23000 rpm for 2 minutes.

### c) Processing conditions

- Inlet main air stream temperature = 210°C
- Inlet main air stream flow rate = 75 Kg/h
- Fluidized bed air stream temperature = 75°C
- Fluidized bed air stream flow rate = 24 Kg/h
- Fine return air stream temperature = 50°C
- Fine return air stream flow rate = 11 Kg/h
- Nozzle gas flow rate = 25 nl/min
- Diameter of the nozzle = 1 mm
- Feed temperature = 40°C

At the beginning of the process the powder mixture was introduced into the chamber via a hopper connected to the fine return pipe. This powder mixture was thus immediately fluidized into the chamber and recycled in closed loop continuously.

After all the temperature settings were obtained in the equipment, the feed was atomized during 10 minutes at a feed flow rate of 0.057 l/min.

The atomization was then stopped and the granules were kept fluidized into the fluidized bed during 10 more minutes. 75 G of dry granules were collected in a glass vessel connected to the fluidized bed via a butterfly valve. The average diameter was 58g >210 microns and 17 g >500 microns.

In order to check that the granules were insoluble in water, 1 g of these granules was introduced into 1000 ml of warm water (60°C). After hydration white granules were clearly visible indicating delayed solubility in water.

The final aspartame load was of about 16 % w/w based on the final weight of the granules. The granule contained on their surface about 20% w/w of a mixture of dry Ca-gluconate and native starch.

## Claims

1. A process for preparing capsules having an average diameter in the range of 100 to 2000 µm and comprising an active ingredient, the process comprising the steps of
a) preparing a solution, suspension and/or emulsion comprising the active ingredient, a carrier or matrix material and a solvent to form a feed, wherein the matrix materials in the feed comprise
polysaccharides, and, preferably saccharides selected from the group of mono- and/or
polysaccharides, and, preferably saccharides selected from the group of mono- and/or disaccharides, trisaccharides and oligosaccharides, or wherein the carrier or matrix material is selected from the group consisting of dextrins, proteins, starch, modified starch, corn syrups, cellulose and derivatives thereof, gums, hydrocolloids, microbial polysaccharides, and mixtures of two or more of these material;;
b) spraying the feed in the form of drops having a mean diameter of 100 to 2000 µm into a spraying tower,
c) introducing, simultaneously to the spraying-step, a powdering agent into the spraying tower, wherein the powdering agent is selected from the group consisting of starch, starch derivatives, talc, bentonite, silicon dioxide, calcium,
magnesium, aluminium or potassium silicates, sodium, potassium or calcium aluminosilicates, calcium, sodium or magnesium carbonates, calcium or magnesium phosphates, ferric ammonium citrate, microcrystalline cellulose and cellulose derivatives or fibres, aluminium, calcium, sodium, magnesium, potassium or ammonium salts of fatty
acids, magnesium oxides and mixtures comprising two or more of these materials, and wherein the powdering agent provides a coating on the surface of the capsules representing 0.1 to 30 wt % of the total weight of the capsules;
d) supplying, simultaneously to the spraying step, a gas having a temperature in the range of -20°C to 500°C into the spraying tower; and,
e) removing the capsules from the spraying tower; wherein, simultaneously with the addition of the powdering agent used in step c), there is introduced into the spraying tower a reactant capable of reacting with said carrier material to modify the dissolution properties of the obtained capsules into aqueous media, wherein the reactant is intended to mean here any compound or substance that is capable of reacting with the carrier or matrix materials of the feed being sprayed in order to induce gelling, polymerisation, cross linking, crystallization or any other chemical or physical transformation of said matrix material susceptible of modifying its water dissolution properties.

2. A process according to claim 1, wherein a hot gas is supplied into the spraying tower, at a temperature of 50°C to 500°C in step d) of the process, preferably 100°C to 250°C, more preferably in the range of 120°C to 200°C.

3. A process according to claim 1, wherein the temperature of the gas is below 30°C, more preferably between -20°C and 10°C.

4. A process according to claim 2, further comprising the step of intercepting the capsules by a fluidised bed after being coated by the powdering agent and after being at least superficially dried by the hot gas in the spraying tower.

5. A process according to claim 1, wherein the solvent is water-based.

6. A process according to claim 1, wherein the powdering agent comprises the reactant capable of reacting with the carrier material of the feed.

7. A process according to claim 1, in which the fluidised bed is supplied with a gas having a temperature in the range of 41 to about 100°C.

8. A process according to claim 1, wherein the feed comprising the active ingredient has a dry matter content in the range of 10 to 80 wt %, preferably in the range 45 - 75 wt %.

9. A process according to claim 1, wherein the capsules have a flavor and/or fragrance ingredient load of 0.1 - 70 wt %, preferably 10 - 50 wt %.

10. A process according to claim 1, wherein the carrier material comprises modified starch, possibly in admixture with maltodextrine and/or a protein, the powdering agent is native starch and the reactant is calcium gluconate.

11. A process according to claim 1, wherein the carrier material comprises polyvinyl alcohol, the powdering agent is native starch and the reactant is disodium sulfate.

12. Capsules obtainable by a process according to any of claims 1 to 11, having an average diameter in the range of 100 to 2000 µm comprising an active ingredient, the load of active ingredient being in the range of 0.1 - 70 wt %, the capsules comprising a coating provided by a powdering agent, whereby the coating provides 0.1 to 30 wt % of the total weight of the capsules.

13. A consumer product comprising the capsules according to claim 12.

14. A consumer product according to claim 13 selected from the group consisting of a perfume, a soap, a cosmetic, another body care product, a hair care product, a food, a beverage, a chewing gum, an oral care product such as a mouthwash or toothpaste, a pharmaceutical and a food supplement.

## Patentansprüche

1. Verfahren für die Herstellung von Kapseln, die einen durchschnittlichen Durchmesser im Bereich von 100 bis 2000 µm aufweisen und einen Wirkstoff umfassen, wobei das Verfahren die Schritte umfasst des
a) Herstellens einer Lösung, Suspension und/oder Emulsion, die den aktiven Bestandteil, ein Träger- oder Matrixmaterial und ein Lösungsmittel umfasst, um ein Einsatzmaterial zu bilden, wobei die Matrixmaterialien in dem Einsatzmaterial Polysaccharide und bevorzugt Saccharide ausgewählt aus der Gruppe von Mono- und/oder Disacchariden, Trisacchariden und Oligosacchariden umfassen, oder wobei das Träger- oder Materixmaterial aus der Gruppe ausgewählt ist bestehend aus Dextrinen, Proteinen, Stärke, modifizierter Stärke, Maissirupen, Cellulose und Derivaten davon, Gummis, Hydrokolloiden, mikrobiellen Polysacchariden und Mischungen von zwei oder mehreren dieser Materialien;
b) Sprühens des Einsatzmaterials in Form von Tropfen, die einen mittleren Durchmesser von 100 bis 2000 µm aufweisen, in einen Sprühturm,
c) Einführens, gleichzeitig mit dem Sprühschritt, eines Pudermittels in den Sprühturm, wobei das Pudermittel aus der Gruppe ausgewählt ist bestehend aus Stärke, Stärkederivaten, Talk, Bentonit, Siliciumdioxid, Calcium-, Magnesium-, Aluminium- oder Kaliumsilicaten, Natrium-, Kalium- oder Calciumaluminosilicaten, Calcium-, Natrium- oder Magnesiumcarbonaten, Calcium- oder Magnesiumphosphaten, Ammoniumeisen(III)citrat, mikrokristalliner Cellulose und Cellulosederivaten oder - fasern, Aluminium-, Calcium-, Natrium-, Magnesium-, Kalium- oder Ammoniumsalzen von Fettsäuren, Magnesiumoxiden und Mischungen umfassend zwei oder mehr dieser Materialien und wobei das Pudermittel eine Beschichtung auf der Oberfläche der Kapseln bereitstellt, die 0,1 bis 30 Gew.-% des Gesamtgewichts der Kapseln darstellt,
d) Lieferns, gleichzeitig mit dem Sprühschritt, eines Gases, das eine Temperatur im Bereich von -20 °C bis 500 °C aufweist, in den Sprühturm; und
e) Entfernens der Kapseln aus dem Sprühturm;
wobei gleichzeitig mit der Zugabe des in Schritt c) verwendeten Pudermittels, ein Reaktand in den Sprühturm eingeführt wird, der in der Lage ist, mit dem Trägermaterial zu reagieren, um die Lösungseigenschaften der erhaltenen Kapseln in einem wässrigen Medium zu modifizieren, wobei der Reaktand hier eine Verbindung oder Substanz bedeuten soll, die in der Lage ist, mit den Träger- oder Matrixmaterialien des Einsatzmaterials, das eingesprüht wird, zu reagieren, um eine Gelbildung, Polymerisation, Vernetzung, Kristallisation oder irgend eine andere chemische oder physikalische Umwandlung des Matrixmaterials, das suszeptibel ist, seine Wasserlösungseigenschaften zu modifizieren, auszulösen.

2. Verfahren nach Anspruch 1, wobei ein heißes Gas in den Sprühturm bei einer Temperatur von 50 °C bis 500 °C, bevorzugt 100 °C bis 250 °C, noch bevorzugter im Bereich von 120 °C bis 200 °C in Schritt d) des Verfahrens eingeliefert wird.

3. Verfahren nach Anspruch 1, wobei die Temperatur des Gases unter 30 °C, noch bevorzugter zwischen -20 °C und 10 °C liegt.

4. Verfahren nach Anspruch 2, des Weiteren den Schritt des Abfangens der Kapseln durch ein Wirbelbett, nachdem sie durch das Pudermittel beschichtet und mindestens oberflächlich durch das heiße Gas im Sprühturm getrocknet worden sind.

5. Verfahren nach Anspruch 1, wobei das Lösungsmittel auf Wasser basiert.

6. Verfahren nach Anspruch 1, wobei das Pudermittel den Reaktand, der in der Lage ist, mit dem Trägermaterial des Einsatzmaterials zu reagieren, umfasst.

7. Verfahren nach Anspruch 1, wobei das Wirbelbett mit einem Gas beliefert wird, das eine Temperatur im Bereich von 41 bis etwa 100 °C aufweist.

8. Verfahren nach Anspruch 1, wobei das Einsatzmaterial, das den aktiven Bestandteil umfasst, einen Trockensubstanzgehalt im Bereich von 10 bis 80 Gew.-%, bevorzugt im Bereich von 45 - 75 Gew.-%, aufweist.

9. Verfahren nach Anspruch 1, wobei die Kapseln eine Aroma- und/oder Geruchsbestandteilladung von 0,1 - 70 Gew.-%, bevorzugt 10 - 50 Gew.-% aufweisen.

10. Verfahren nach Anspruch 1, wobei das Trägermaterial modifizierte Stärke, möglicherweise in Mischung mit Maltodextrin und/oder einem Protein umfasst, wobei das Pudermittel native Stärke und der Reaktand Calciumgluconat ist.

11. Verfahren nach Anspruch 1, wobei das Trägermaterial Polyvinylalkohol umfasst, das Pudermittel native Stärke ist und der Reaktand Dinatriumsulfat ist.

12. Kapseln, die durch ein Verfahren nach einem der Ansprüche 1 bis 11 erhältlich sind und einen durchschnittlichen Durchmesser im Bereich von 100 bis 2000 µm aufweisen und einen aktiven Bestandteil umfassen, wobei die Beladung von aktivem Bestandteil im Bereich von 0,1 - 70 Gew.-% liegt, wobei die Kapseln eine Beschichtung umfassen, die durch ein Pudermittel bereitgestellt wird, wodurch die Beschichtung 0,1 bis 30 Gew.-% des Gesamtgewichts der Kapseln bereitstellt.

13. Konsumartikel umfassend die Kapseln nach Anspruch 12.

14. Konsumartikel nach Anspruch 13 ausgewählt aus der Gruppe bestehend aus einem Parfüm, einer Seife, einem Kosmetikum, einem anderen Köperpflegeprodukt, einem Haarpflegeprodukt, einem Nahrungsmittel, einem Getränk, einem Kaugummi, einem Mundpflegeprodukt wie beispielsweise einem Mundwasser oder einer Zahnpasta, einem Pharmazeutikum und einem Nahrungsergänzungsmittel.

## Revendications

1. Procédé de préparation de capsules ayant un diamètre moyen dans la plage de 100 à 2000 µm et comprenant un principe actif, le procédé comprenant les étapes consistant à:
a) préparer une solution, suspension et/ou émulsion comprenant le principe actif, une matière véhicule ou matricielle et un solvant pour former une charge, où les matières matricielles dans la charge comprennent des polysaccharides et des saccharides sélectionnés de préférence parmi le groupe de monosaccharides et/ou disaccharides, trisaccharides et oligosaccharides, ou bien où la matière véhicule ou matricielle est sélectionnée parmi le groupe consistant en dextrines, protéines, amidon, amidon modifié, sirops de maïs, cellulose et ses dérivés, gommes, hydrocolloïdes, polysaccharides microbiens et des mélanges de deux de ces matières ou plus;
b) atomiser la charge sous forme de gouttes ayant un diamètre moyen de 100 à 2000 µm dans une tour d'atomisation;
c) introduire, en même temps que l'étape d'atomisation, un agent de pulvérisation dans la tour d'atomisation, où l'agent de pulvérisation est sélectionné parmi le groupe consistant en amidon, dérivés d'amidon, talc, bentonite, dioxyde de silicium, silicates de calcium, de magnésium, d'aluminium ou de potassium, aluminosilicates de sodium, de potassium ou de calcium, carbonates de calcium, de sodium ou de magnésium, phosphates de calcium ou de magnésium, citrate d'ammonium ferrique, cellulose microcristalline et dérivés ou fibres de cellulose, sels d'aluminium, de calcium, de sodium, de magnésium, de potassium ou d'ammonium d'acides gras, oxydes de magnésium et des mélanges comprenant deux de ces matières ou plus, et où l'agent de pulvérisation fournit un enrobage sur la surface des capsules représentant 0,1 à 30 % en poids du poids total des capsules;
d) fournir, en même temps que l'étape d'atomisation, un gaz ayant une température dans la plage de -20 °C à 500 °C dans la tour d'atomisation, et
e) retirer les capsules de la tour d'atomisation;
où, en même temps que l'addition de l'agent de pulvérisation utilisé à l'étape c), un réactif capable de réagir avec ladite matière véhicule pour modifier les propriétés de dissolution des capsules obtenues dans des milieux aqueux, est introduit dans la tour d'atomisation, où le réactif est destiné à signifier ici n'importe quel composé ou substance qui est capable de réagir avec les matières véhicules ou matricielles de la charge étant atomisée, afin d'induire une gélification, polymérisation, réticulation, cristallisation ou n'importe quelle autre transformation chimique ou physique de ladite matière matricielle susceptible de modifier ses propriétés de dissolution dans l'eau.

2. Procédé selon la revendication 1, dans lequel un gaz chaud est fourni dans la tour d'atomisation à l'étape d) du procédé à une température de 50 °C à 500 °C, de préférence de 100 °C à 250 °C, plus préférentiellement dans la plage de 120 °C à 200 °C.

3. Procédé selon la revendication 1, dans lequel la température du gaz est inférieure à 30 °C, plus préférentiellement entre -20 °C et 10 °C.

4. Procédé selon la revendication 2, comprenant en outre l'étape consistant à intercepter les capsules avec un lit fluidisé après qu'elles ont été enrobées par l'agent de pulvérisation et après qu'elles ont été séchées au moins superficiellement par le gaz chaud dans la tour d'atomisation.

5. Procédé selon la revendication 1, dans lequel le solvant est à base d'eau.

6. Procédé selon la revendication 1, dans lequel l'agent de pulvérisation comprend le réactif capable de réagir avec la matière véhicule de la charge.

7. Procédé selon la revendication 1, dans lequel le lit fluidisé est alimenté avec un gaz ayant une température dans la plage de 41 à environ 100 °C.

8. Procédé selon la revendication 1, dans lequel la charge comprenant le principe actif a une teneur en matière sèche dans la plage de 10 à 80 % en poids, de préférence dans la plage de 45 - 75 % en poids.

9. Procédé selon la revendication 1, dans lequel les capsules ont une charge d'ingrédient aromatique et/ou parfumant de 0,1 - 70 % en poids, de préférence de 10- 50 % en poids.

10. Procédé selon la revendication 1, dans lequel la matière véhicule comprend de l'amidon modifié, éventuellement en mélange avec de la maltodextrine et/ou une protéine, l'agent de pulvérisation est de l'amidon naturel et le réactif est du gluconate de calcium.

11. Procédé selon la revendication 1, dans lequel la matière véhicule comprend de l'alcool polyvinylique, l'agent de pulvérisation est de l'amidon naturel et le réactif est du sulfate de disodium.

12. Capsules pouvant être obtenues par un procédé selon l'une quelconque des revendications 1 à 11, ayant un diamètre moyen dans la plage de 100 à 2000 µm, comprenant un principe actif, la charge de principe actif étant dans la plage de 0,1 - 70 % en poids, les capsules comprenant un enrobage fourni par un agent de pulvérisation, moyennant quoi l'enrobage fournit 0,1 à 30 % en poids du poids total des capsules.

13. Bien de consommation comprenant les capsules selon la revendication 12.

14. Bien de consommation selon la revendication 13, sélectionné parmi le groupe consistant en un parfum, un savon, un cosmétique, un autre produit de soin corporel, un produit de soin capillaire, un aliment, une boisson, une gomme à mâcher, un produit de soin buccal tel qu'un bain de bouche ou une pâte dentifrice, un produit pharmaceutique et un supplément alimentaire.
